# EUROPEAN PATENT APPLICATION

(11) **EP 2 520 218 A1**
(43) Date of publication of application: **07.11.2012**
(21) Application number: 12162806.9
(22) Date of filing: 09.02.2007
(51) Int. Cl.: A61B 1/07, A61B 1/05, A61B 1/012, G02B 27/28

(54) **Endoscope assembly with a polarizing filter**

(30) Priority: 09.02.2006 US 772442 P; 12.12.2006 US 609838; 23.01.2007 US 626189; 06.02.2007 US 672020
(62) Divisional of application: 07717235.1
(71) Applicant: Avantis Medical Systems, Inc., Sunnyvale, CA 94085-4511 (US)
(72) Inventor: Bayer, Lex, Palo Alto, California 94306 (US); Desai, Rupesh, San Jose, California 95132 (US); Neil, Alex, Daly City, California 94015 (US)
(74) Representative: ZBM Patents

(57) **Abstract**

Endoscope assemblies comprising image sensor(s), light source(s) and polarizing filter(s).

## Description

This application claims the benefit of United States Provisional Patent Application No. 60/772,442, filed February 9, 2006, the entire disclosure of which is incorporated herein by reference.

This application claims the benefit of United States Patent Application No. 11/672,020, filed February 6, 2007, the entire disclosure of which is incorporated herein by reference.

This application claims the benefit of United States Patent Application No. 11/626,189, filed January 23, 2007, the entire disclosure of which is incorporated herein by reference.

This application claims the benefit of United States Patent Application No. 11/609,838, filed December 12, 2006, the entire disclosure of which is incorporated herein by reference.

This application is a continuation-in-part application of United States Patent Application No. 11/215,660, filed August 29, 2005, the entire disclosure of which is incorporated herein by reference.

This application is a continuation-in-part application of United States Patent Application No. 11/030,559, filed January 5, 2005, the entire disclosure of which is incorporated herein by reference.

### TECHNICAL FIELD OF THE INVENTION

The present invention relates to an endoscope assembly with a polarizing filter.

### BACKGROUND OF THE INVENTION

A conventional endoscope is a medical device comprising a flexible tube, and a camera and a light source mounted on the distal end of the flexible tube. The endoscope is insertable into an internal body cavity through a body orifice to examine the body cavity and tissues for diagnosis. The tube of the endoscope has one or more longitudinal channels, through which an instrument can reach the body cavity to take samples of suspicious tissues or to perform other surgical procedures such as polypectomy.

There are many types of endoscopes, and they are named in relation to the organs or areas with which they are used. For example, gastroscopes are used for examination and treatment of the esophagus, stomach and duodenum; colonoscopes for the colon; bronchoscopes for the bronchi; laparoscopes for the peritoneal cavity; sigmoidoscopes for the rectum and the sigmoid colon; arthroscopes for joints; cystoscopes for the urinary bladder; and angioscopes for the examination of blood vessels.

Each endoscope has a single forward viewing camera mounted at the distal end of the flexible tube to transmit an image to an eyepiece or video camera at the proximal end. The camera is used to assist a medical professional in advancing the endoscope into a body cavity and looking for abnormalities. The camera provides the medical professional with a two-dimensional view from the distal end of the endoscope. To capture an image from a different angle or in a different portion, the endoscope must be repositioned or moved back and forth. Repositioning and movement of the endoscope prolongs the procedure and causes added discomfort, complications, and risks to the patient. Additionally, in an environment similar to the lower gastro-intestinal tract, flexures, tissue folds and unusual geometries of the organ may prevent the endoscope's camera from viewing all areas of the organ. The unseen area may cause a potentially malignant (cancerous) polyp to be missed.

This problem can be overcome by providing an auxiliary camera and an auxiliary light source. The auxiliary camera and light source can be oriented to face the main camera and light source, thus providing an image of areas not viewable by the endoscope's main camera. This arrangement of cameras and light sources can provide both front and rear views of an area or an abnormality. In the case of polypectomy where a polyp is excised by placing a wire loop around the base of the polyp, the camera arrangement allows better placement of the wire loop to minimize damage to the adjacent healthy tissue.

Since the main camera and light source face the auxiliary camera and light source, the main light source interferes with the auxiliary camera, and the auxiliary light source interferes with the main camera. Light interference is the result of the light from a light source being projected directly onto the lens of a camera. This may cause light glare, camera blooming, or over saturation of light, resulting in inferior image quality.

Additionally, because of space constraint, the auxiliary camera and auxiliary light source are typically smaller than the main camera and main light source and use different technologies. Different types of cameras often require different levels of illumination. For example, the main camera generally requires a higher level of illumination and needs a more powerful light source. As a result, the auxiliary camera is often exposed to a significant amount of glare caused by the powerful main light source. Therefore, there is a need to reduce or prevent light interference between the main camera and main light source and the auxiliary camera and auxiliary light source.

### SUMMARY OF THE INVENTION

It is an object of this invention to address the problem of light interference involving the use of imaging devices and light sources for endoscopes. According to one aspect of the invention, the solution lies in the use of one or more polarizing filters including one or more linear or circular polarizing filters.

In accordance with one aspect of the invention, an endoscope assembly includes an imaging device, a light source, and a polarizing filter disposed in front of the light source. Preferably, the light source is positioned to illuminate a field of view of the imaging device. In a preferred embodiment, the endoscope assembly further includes a second polarizing filter disposed in front of the imaging device. Preferably, the planes of polarization of the first and second polarizing filters are aligned. A polarizing filter may be disposed on a cap or in a lens assembly of the imaging device.

In accordance with another aspect of the invention, an endoscope assembly includes an imaging device, a light source, and a polarizing filter disposed in front of the imaging device.

In accordance with still another aspect of the invention, an endoscope assembly includes an imaging device, a light source, and a polarizing filter disposed in front of both the imaging device and the light source.

In accordance with yet another aspect of the invention, an endoscope assembly includes an imaging device, a first polarizing filter disposed in front of the imaging device, a light source; and a second polarizing filter disposed in front of the light source. The plane of polarization of the first polarizing filter is at a substantially 90° angle from the plane of polarization of the second polarizing filter. Preferably, the light source faces the imaging device so that light from the light source is projected onto the imaging device.

In a preferred embodiment, the endoscope assembly further includes a second imaging device, a third polarizing filter disposed in front of the second imaging device, a second light source, and a fourth polarizing filter disposed in front of the second light source. The plane of polarization of the third polarizing filter is at a substantially 90° angle from the plane of polarization of the fourth polarizing filter.

In another preferred embodiment, the first light source faces the first imaging device, and the second light source faces the second imaging device.

In a further preferred embodiment, the second light source is positioned to illuminate a field of view of the first imaging device, and the first light source is positioned to illuminate a field of view of the second imaging device.

Preferably, the first imaging device and second light source are mounted on an insertion tube, and the second imaging device and first light source are mounted on an imaging assembly. The insertion tube may a channel, and a portion of the imaging assembly may be rotatably disposed inside the channel of the insertion tube with the second imaging device and first light source extending beyond an end of the insertion tube.

In a preferred embodiment, the endoscope assembly includes a locking mechanism that rotationally locks the insertion tube and imaging assembly. In accordance with still yet another aspect of the invention, an endoscope assembly includes an insertion tube having a channel, an imaging assembly, and a locking mechanism. A portion of the imaging assembly is rotatably disposed inside the channel of the insertion tube, and the locking mechanism rotationally locks the insertion tube and imaging assembly.

In accordance with a further aspect of the invention, a major-minor endoscope includes a major endoscope having a first light source positioned proximate to one end and having a first imaging device positioned proximate to the one end, the major endoscope further including a channel along its length, a minor endoscope adapted for insertion into and through the channel of the major endoscope, the minor endoscope including an elongated body, a second light source positioned proximate to one end and having a second imaging device positioned proximate to the one end, a first polarizer positioned to polarize the light from the light source of one of the endoscopes, and a second polarizer positioned to polarize light received by the imaging device of the other endoscope. Preferably, the first polarizer is positioned to polarize the light from the light source of the major endoscope and the second polarizer is positioned to polarize the light received by the imaging device of the minor endoscope. Additionally, the first polarizer may be positioned to polarize the light from the light source of the minor endoscope and the second polarizer may be positioned to polarize the light received by the imaging device of the major endoscope. Furthermore, the first polarizer may be positioned to polarize the light from the light source of the major endoscope and the second polarizer may be positioned to polarize the light received by the imaging device of the minor endoscope. Preferably, the major-minor endoscope may further include a third polarizer positioned to polarize the light from the light source of the major endoscope and a fourth polarizer positioned to polarize the light received by the imaging device of the minor endoscope.

In accordance with a still further aspect of the invention, an endoscope includes an elongated body having a first imaging device positioned proximate to one end; and a first polarizer positioned to polarize light received by the imaging device.

In accordance with a yet further aspect of the invention, an endoscope and independently positionable polarized light source include the above endoscope and further include an independently positionable light source adapted for use within the human body. The light source may include an elongated body, a first light source proximate to one end, and a polarizer positioned to polarize the light emitted from the light source.

A yet still further aspect of the invention is directed to a pair of polarized endoscopes adapted for cooperative use within the human body. Each endoscope comprises an elongated body, an imaging device positioned proximate to one end, and a light source positioned proximate to the one end. A polarizer is positioned to polarize the light from the light source of one of the endoscopes and a second polarizer is positioned to polarize the light received by the imaging device of the other endoscope. The pair may further include a third polarizer positioned to polarize the light from the light source of the other endoscope and a fourth polarizer positioned to polarize the light received by the imaging device of the one endoscope.

### BRIEF DESCRIPTION OF THE DRAWINGS

Figure 1 shows a perspective view of an endoscope with an imaging assembly according to one embodiment of the present invention.
Figure 2 shows a perspective view of the distal end of an insertion tube of the endoscope of Figure 1 with a polarizer cap.
Figure 3 shows a perspective back view of the polarizer cap of Figure 2.
Figure 4 shows a perspective view of the imaging assembly shown in Figure 1.
Figure 5 shows a perspective view of the distal ends of the endoscope and imaging assembly of Figure 1 with a cross-sectional view of the lens barrel of the imaging assembly.
Figure 6 shows a cross-sectional view of a peritoneal cavity with multiple endoscopes.

### DETAILED DESCRIPTION OF EMBODIMENTS OF THE INVENTION

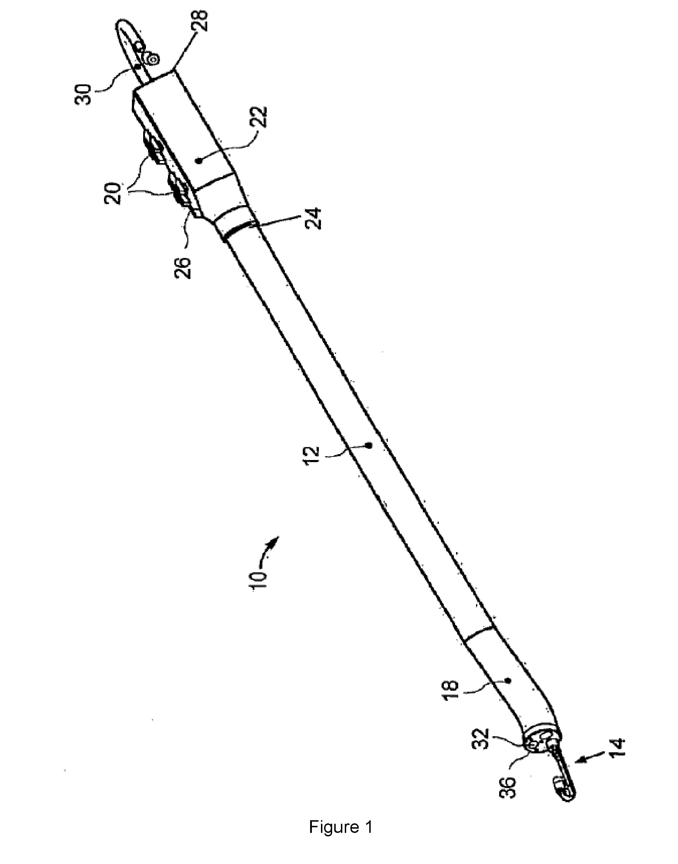

Figure 1 illustrates a first exemplary endoscope 10 of the present invention.

This endoscope 10 can be used in a variety of medical procedures in which imaging of a body tissue, organ, cavity or lumen is required. The types of procedures include, for example, anoscopy, arthroscopy, bronchoscopy, colonoscopy, cystoscopy, EGD, laparoscopy, and sigmoidoscopy.

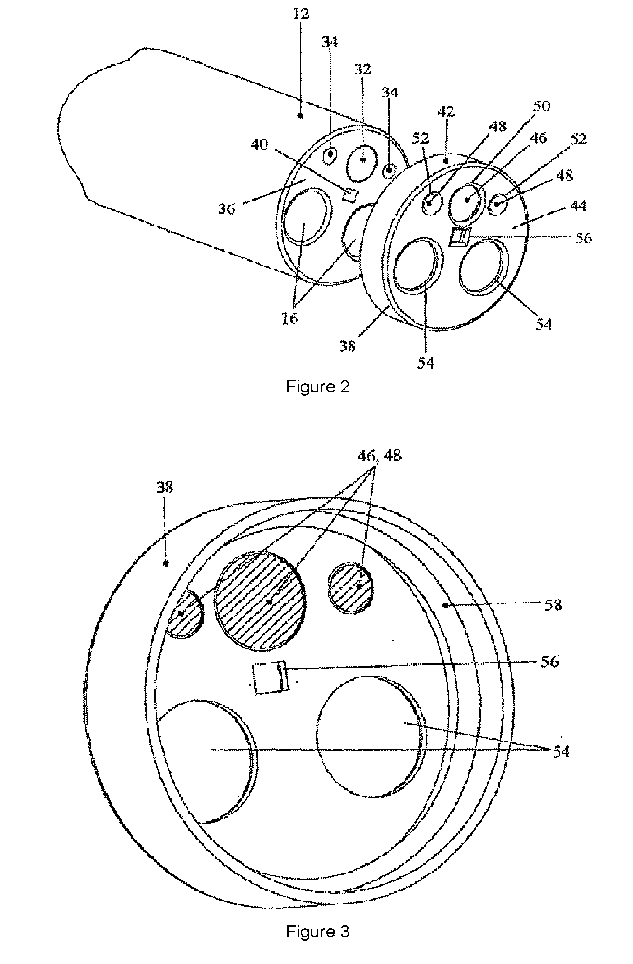

The endoscope 10 of Figure 1 includes an insertion tube 12 and an imaging assembly 14, a section of which is housed inside the insertion tube 12. As shown in Figure 2, the insertion tube 12 has two longitudinal channels 16. In general, however, the insertion tube 12 may have any number of longitudinal channels. An instrument can reach the body cavity to perform any desired procedures, such as to take samples of suspicious tissues or to perform other surgical procedures such as polypectomy. The instruments may be, for example, a retractable needle for drug injection, hydraulically actuated scissors, clamps, grasping tools, electrocoagulation systems, ultrasound transducers, electrical sensors, heating elements, laser mechanisms and other ablation means. In some embodiments, one of the channels can be used to supply a washing liquid such as water for washing. Another or the same channel may be used to supply a gas, such as CO2 or air into the organ. The channels 16 may also be used to extract fluids or inject fluids, such as a drug in a liquid carrier, into the body. Various biopsy, drug delivery, and other diagnostic and therapeutic devices may also be inserted via the channels 16 to perform specific functions.

The insertion tube 12 preferably is steerable or has a steerable distal end region 18 as shown in Figure 1. The length of the distal end region 18 may be any suitable fraction of the length of the insertion tube 12, such as one half, one third, one fourth, one sixth, one tenth, or one twentieth. The insertion tube 12 may have control cables (not shown) for the manipulation of the insertion tube 12. Preferably, the control cables are symmetrically positioned within the insertion tube 12 and extend along the length of the insertion tube 12. The control cables may be anchored at or near the distal end 36 of the insertion tube 12. Each of the control cables may be a Bowden cable, which includes a wire contained in a flexible overlying hollow tube. The wires of the Bowden cables are attached to controls 20 in the handle 22. Using the controls 20, the wires can be pulled to bend the distal end region 18 of the insertion tube 12 in a given direction. The Bowden cables can be used to articulate the distal end region 18 of the insertion tube 12 in different directions.

As shown in Figure 1, the endoscope 10 may include a control handle 22 connected to the proximal end 24 of the insertion tube 12. Preferably, the control handle 22 has one or more ports and/or valves (not shown) for controlling access to the channels 16 of the insertion tube 12. The -ports and/or valves can be air or water valves, suction valves, instrumentation ports, and suction/instrumentation ports. As shown in Figure 1 , the control handle 22 may additionally include buttons 26 for taking pictures with an imaging device on the insertion tube 12, the imaging assembly 14, or both.

The proximal end 28 of the control handle 22 may include an accessory outlet 30 (Figure 1) that provides fluid communication between the air, water and suction channels and the pumps and related accessories. The same outlet 30 or a different outlet can be used for electrical lines to light and imaging components at the distal end of the endoscope 10.

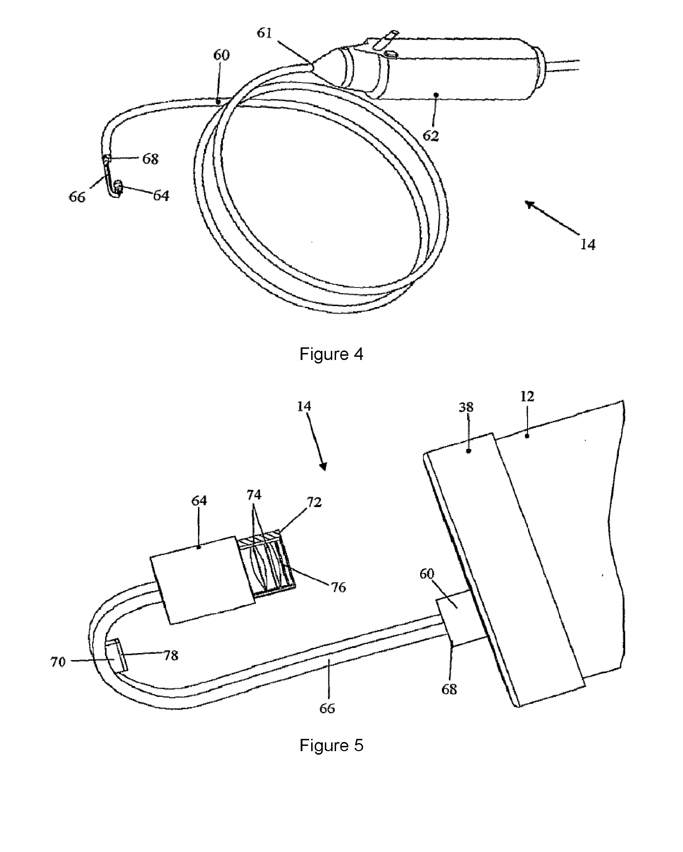

As shown in Figure 2, the endoscope 10 also includes a main imaging device 32 and main light sources 34, both of which are disposed at the distal end 36 of the insertion tube 12, and a polarizer cap 38 that is adapted to be mounted on the distal end 36 of the insertion tube 12 to cover the main imaging device 32 and main light sources 34. Figure 2 shows the polarizer cap 38 removed from the distal end 36 of the insertion tube 12, and Figure 5 shows the polarizer cap 38 mounted on the distal end 36 of the insertion tube 12.

The main imaging device 32 at the distal end 36 of the insertion tube 12 may include, for example, a lens, single chip sensor, multiple chip sensor or fiber optic implemented devices. The main imaging device 32, in electrical communication with a processor and/or monitor, may provide still images or recorded or live video images. The light sources 34 may be light emitting diodes (LEDs) or fiber optical delivery of light from an external light source. The light sources 34 preferably are equidistant from the main imaging device 32 to provide even illumination. The intensity of each light source 34 can be adjusted to achieve optimum imaging. The circuits for the main imaging device 32 and light sources 34 may be incorporated into a printed circuit board (PCB). As shown in Figure 2, the insertion tube 12 has a channel 40 for supplying a liquid such as water for cleaning the lenses of the main imaging device 32 and the light sources 34.

The polarizer cap 38, as shown in Figures 2 and 3, includes a cylindrical sidewall 42, an end wall 44, and polarizing filters 46, 48 mounted on the end wall 44. The cylindrical sidewall 42 and end wall 44 may form an integral part that is made by injection molding of a suitable biocompatible material such as medical grade plastics. The end wall 44 preferably has an opening 50 for accommodating the polarizing filter 46 for the main imaging device 32. The opening 50 may have any arrangement suitable for retaining the polarizing filter 46. For example, the opening 50 may have a recessed lip for receiving the polarizing filter 46. The polarizing filter 46 can be placed in the recessed lip and fixed there by adhesive bonding or by a mechanical snap fit.

The end wall 44 preferably has an opening 52 for accommodating the polarizing filter 48 for each of the light sources 34. The opening 52 may have any arrangement suitable for retaining the polarizing filter 48. One example of such suitable arrangement is the recessed lip described above. The end wall 44 preferably has an opening 54 for each of the instrument channels 16 so that the cap 38 does not block the channels 16.

The end wall 44 may further include an opening 56 for the channel 40 for supplying a liquid to clean the lenses of the imaging device 32 and the light sources 34. Preferably, the cap 38 has one or more features that allow liquid from the channel 40 to reach over the cap 38 to clean the exterior surfaces of polarizing filters 46, 48. For example, the end wall 44 of the cap 38 may be sufficiently thin to allow the liquid from the channel 40 to reach over the cap 38 to clean the exterior surfaces of polarizing filters 46, 48. Alternatively, the cap 38 may have variable thickness and/or angled features that allow liquid from the channel 40 to reach the polarizing filters 46, 48. Furthermore, the cap 38 may have a ramp, plate or channel that allows liquid from the channel 40 to reach the polarizing filters 46, 48. The locations, configurations and sizes of the openings 50, 51, 54, 56 preferably correspond to the locations, configurations and sizes of the main imaging device 32, light sources 34, channels 16, and clean liquid channel 40, respectively.

As shown in Figure 3, the cap 38 preferably has a ring 58 located around the inner perimeter of the cap 38. The ring 58 helps secure the cap 38 to the distal end region of the insertion tube 12. In a preferred embodiment, the ring 58 is made from a compressive material such as silicon. Alternatively, the ring 58 can be made from other compressive materials, such as compressive rubbers, polymers and/or foams. The ring 58 may be attached the inner perimeter of the cap 38 by any suitable means such as adhesive bonding, mechanical over molding, or plastic snap features.

The inside diameter of the ring 58 preferably is slightly smaller than the outer diameter of the insertion tube 12 so that the ring 58 can apply a compressive force to the outer surface of the insertion tube 12. This compressive force preferably is sufficient to create the necessary friction force to ensure that the cap 38 remains in the same position and orientation during a medical procedure, yet to allow the cap 38 to be slide on and off of the insertion tube 12 without difficulty.

Alternatively, the cap 38 may have any other type of arrangement for attachment to the insertion tube 12. For example, the cap 38 may have clasps which snap on to the insertion tube 12. In some embodiments, the attachment may be similar to the way in which a suction cap for endoscopic mucosal resection is attached to a colonoscope, as is well known in the art.

The terms "polarizing filter" and "polarizer" as used in this specification refer to any device that blocks one or more components of light while allowing one or more other components to pass through. In some cases, polarizing filters may be made from a material that blocks light waves traveling in all planes from passing through the filter except for light waves propagating in one specific plane of orientation, often referred to as the plane of polarization or the plane of transmission. Polarizing filters may be constructed using various techniques that use light absorption, reflection, scattering or birefringence to block light from passing through the filter that is not orientated parallel with the plane of transmission.

When one polarizing filter is placed in front of another polarizing filter and noncoherent natural white light is passed through the two polarizing filters, the amount of light that passes through the two polarizing filters is proportional to the relative angle of orientation of the two filters. This is because when the polarization plane of the two filters is at the same angle of orientation, the majority of light waves in the plane of transmission will pass through both filters. As one of the filters is rotated, light that is polarized by the first filter is then attenuated or blocked by the second filter. The maximum amount of light reduction or extinction occurs when the polarizing planes of the two filters are orientated at 90° relative to each other. It is common to find polarizing filters that when orientated at 90° provide 99% or greater extinction of light transmission.

As shown in Figures 4 and 5, the imaging assembly 14 may include a tubular body 60, a handle 62 connected to the proximal end 61 of the tubular body 60, an auxiliary imaging device 64, a link 66 that provides physical and/or electrical connection between the auxiliary imaging device 64 to the distal end 68 of the tubular body 60, and an auxiliary light source 70 (Figure 5).

As shown in Figure 5, the imaging assembly 14 is used to provide an auxiliary imaging device at the distal end of the endoscope 10. To this end, the imaging assembly 14 is placed inside one of the channels 16 of the endoscope's insertion tube 12 with its auxiliary imaging device 64 disposed beyond the distal end 36 of the insertion tube 12. This can be accomplished by first inserting the distal end of the imaging assembly 14 into the insertion tube's channel 16 from the endoscope's handle 18 and then pushing the imaging assembly 14 further into the assembly 14 until the auxiliary imaging device 64 and link 66 of the imaging assembly 14 are positioned outside the distal end 36 of the insertion tube 12 as shown in Figure 5.

As shown in Figure 5, the auxiliary imaging device 64 may include a lens barrel 72 having one or more lenses 74, an imaging sensor, and a printed circuit board (PCB). The imaging sensor may be an electronic device which converts light incident on photosensitive semiconductor elements into electrical signals. The imaging sensor may detect either color or black-and-white images. The signals from the imaging sensor can be digitized and used to reproduce an image that is incident on the imaging sensor. Two commonly used types of image sensors are Charge Coupled Devices (CCD) such as a VCC-5774 produced by Sanyo of Osaka, Japan and Complementary Metal Oxide Semiconductor (CMOS) camera chips such as an OVT 6910 produced by OmniVision of Sunnyvale, California. The endoscope 10 preferably includes a polarizing filter 76 placed in front of the auxiliary imaging device 64. The polarizing filter 16 may be placed inside the lens barrel 72. Alternatively, the polarizing filter 76 may be placed directly onto the image sensor itself, or incorporated at various other locations in the lens barrel 72 such as at the end closest to the imaging sensor, or even between the lenses 74. Furthermore, the polarizing filter 76 may be simply placed in front of the auxiliary imaging device.

When the imaging assembly 14 is properly installed in the insertion tube 12, the auxiliary imaging device 64 of the imaging assembly 14 preferably faces backwards towards the main imaging device 32 as illustrated in Figure 3. The auxiliary imaging device 64 may be oriented so that the auxiliary imaging device 64 and the main imaging device 32 have adjacent or overlapping viewing areas. Alternatively, the auxiliary imaging device 64 may be oriented so that the auxiliary imaging device 64 and the main imaging device 32 simultaneously provide different views of the same area. Preferably, the auxiliary imaging device 64 provides a retrograde view of the area, while the main imaging device 32 provides a front view of the area. However, the auxiliary imaging device 64 could be oriented in other directions to provide other views, including views that are substantially parallel to the axis of the main imaging device 32.

As shown in Figures 2 and 3, the link 66 connects the auxiliary imaging device 64 to the distal end 68 of the tubular body 60. Preferably, the link 66 is a flexible link that is at least partially made from a flexible shape memory material that substantially tends to return to its original shape after deformation. Shape memory materials are well known and include shape memory alloys and shape memory polymers. A suitable flexible shape memory material is a shape memory alloy such as nitinol. The flexible link 66 is straightened to allow the distal end of the imaging assembly 14 to be inserted into the proximal end of assembly 14 of the insertion tube 12 and then pushed towards the distal end 36 of the insertion tube 12. When the auxiliary imaging device 64 and flexible link 66 are pushed sufficiently out of the distal end 36 of the insertion tube 12, the flexible link 66 resumes its natural bent configuration as shown in Figure 3. The natural configuration of the flexible link 66 is the configuration of the flexible link 66 when the flexible link 66 is not subject to any force or stress. When the flexible link 66 resumes its natural bent configuration, the auxiliary imaging device 64 faces substantially back towards the distal end 36 of the insertion tube 12 as shown in Figure 5.

In the illustrated embodiment, the auxiliary light source 70 (as well as other components) of the imaging assembly 14 is placed on the flexible link 66, in particular on the curved concave portion of the flexible link 66. The auxiliary light source 70 provides illumination for the auxiliary imaging device 64 and may face substantially the same direction as the auxiliary imaging device 64 as shown in Figure 5.

The endoscope 10 includes another polarizing filter 78 placed in front of the auxiliary light source 70. The polarizing filter 78 may be attached to the auxiliary light source 70 by any suitable means such as adhesive bonding or welding.

The flexible link 66 may be encapsulated or shrouded by flexible tubing, heat-shrinkable tubing, urethanes, rubber or silicon so as to allow smooth profile transition from the tubular body 60 to the imaging device 64. This encapsulation may be translucent to allow light from the light source 70 to project through the encapsulation, or the encapsulation may include a window section around the light source 70.

Since the main imaging device 32 and its light source 34 face the auxiliary imaging device 64 and its light source 70, the light sources 34, 45 of the imaging devices 32, 64 may interfere with the opposing imaging device 64, 32. That is, the main light source 34 may shine directly into auxiliary imaging device 64 and the auxiliary light source 70 may shine directly into the main imaging device 32, degrading both images.

To eliminate or reduce the light interference, the polarization plane of the polarizing filter 46 for the main imaging device 32 may be set at a substantially 90° angle from the polarization plane of the polarizing filter 78 for the auxiliary light source 70. With this arrangement, the light, which is emitted from the auxiliary light source 70 and passes though the polarizing filter 78, may be filtered out by the polarizing filter 46 and may not reach the main imaging device 32. Additionally or alternatively, the polarization plane of the polarizing filter 76 for the auxiliary imaging device 64 may be set at a substantially 90° angle from the polarization plane of the polarizing filters 48 for the main light sources 34. With this arrangement, the light, which is emitted from the main light sources 34 and passes though the polarizing filters 48, may be filtered out by the polarizing filter 76 and may not reach the auxiliary imaging device 64.

Moreover, to provide illumination, the polarization plane of the polarizing filter 46 for the main imaging device 32 may be substantially, aligned with the polarization plane of the polarizing filters 48 for the main light sources 34 so that the light, which is emitted from the main light sources 34 and passes though the polarizing filters 48, may pass through the polarizing filter 46 and may be received by the main imaging device 32. Additionally or alternatively, the polarization plane of the polarizing filter 76 for the auxiliary imaging device 64 may be substantially aligned with the polarization plane of the polarizing filter 78 for the auxiliary light source 70 so that the light, which is emitted from the auxiliary light source 70 and passes though the polarizing filter 78, may pass through the polarizing filter 76 and may be received by the auxiliary imaging device 64.

The desired relative orientations of the polarizing filters' the polarization planes, as set forth above, may be achieved in any suitable manner. For example, the polarization planes of the polarizing filters 46, 48 for the main imaging device 32 and main light sources 34 may be aligned and fixed in the polarizer cap 38, and the polarization planes of the polarizing filters 76, 78 for the auxiliary imaging device 64 and auxiliary light source 70 may be aligned and fixed in the imaging assembly 14. Then the imaging assembly 14 may be rotated within the channel 16 of the insertion tube 12 by means of its handle 62 until the polarization planes of the polarizing filters 76,78 in the imaging assembly 14 are at a substantially 90° angle from the polarization planes of the polarizing filters 46, 48 in the polarizer cap 38.

The orientations of the polarizing filters' the polarization planes may be determined and set during attachment by viewing a light with a known polarization passing through polarizing filters. Alternatively, the polarizing filters may have asymmetrical shapes or other locating features so that the orientations of their polarization planes may be readily determined.

The auxiliary imaging device 64 and its light source 70 may be connected to a control box (not shown) via electrical conductors that extend from the imaging device 64 and light source 70; through the link 66, tubular body 60, and handle 62; to the control box. The electrical conductors may carry power and control commands to the auxiliary imaging device 64 and its light source 70 and image signals from the auxiliary imaging device 64 to the control box.

The control box includes at least an image and signal processing device and a housing in which the image and signal processing device is disposed, although the control box can be configured in any suitable manner. The housing may include a control panel and connectors. The control panel includes buttons and knobs for controlling the functionalities of the control box.

The image and signal processing device may include one or more integrated circuits and memory devices along with associated discrete components. The device allows image signals from the imaging devices 32, 64 to be processed for the enhancement of image quality, extraction of still images from the image signals, and conversion of video format for compatibility with the display device.

The control box preferably processes the video image signal from the auxiliary imaging device 64 and transmits it to a display device such as a television or a monitor such as a liquid crystal display monitor. Still images can be captured from the video image signal. The video image or still image may be displayed on the display device. The display device may also include textual data that are used to display information such as patient information, reference numbers, date, and/or time.

The image signal from the main imaging device 32 may also be processed by the control box in the same way that the image signal from the auxiliary imaging device 64 is processed. The images from the main and auxiliary imaging devices 32, 64 may be displayed on two separate monitors or on the same monitor with a split screen.

The control box may further be used to adjust the parameters of the imaging devices 32, 64 and their light sources 34, 70, such as brightness, exposure time and mode settings. The adjustment can be done by writing digital commands to specific registers controlling the parameters. The registers can be addressed by their unique addresses, and digital commands can be read from and written to the registers to change the various parameters. The control box can change the register values by transmitting data commands to the registers.

The control box may additionally be used as an interface to the patient records database. A large number of medical facilities now make use of electronic medical records. During the procedure relevant video and image data may need to be recorded in the patient electronic medical records (EMR) file. The signal processing circuit can convert image and video data to a format suitable for filing in the patient EMR file such as images in .jpeg, tif, or .bmp format among others. The processed signal can be transmitted to the medical professional's computer or the medical facilities server via a cable or dedicated wireless link. A switch on the control panel can be used to enable this transmission. Alternatively the data can be stored with a unique identification for the patient in electronic memory provided in the control box itself. The signal processing circuit can be utilized to convert the video and image data to be compatible with the electronic medical records system used by the medical professional. The processing may include compression of the data. A cable or a wireless link may be used to transmit the data to a computer.

During endoscopy, a technician may first install the polarizer cap 38 onto the endoscope's insertion tube 12. A physician may then insert the endoscope into a body cavity through an orifice of the body. Once the endoscope is inserted, the physician may decide to use the imaging assembly 14 in order to obtain a rear-viewing image of a certain tissue. The physician may straighten the flexible link 66 of the imaging assembly 14 and insert the straightened distal end of the imaging assembly 14 into the channel 16 of the endoscope's insertion tube 12 from the handle 22. The imaging assembly 14 can then be pushed towards the distal end 36 of the insertion tube 12. When the auxiliary imaging device 64 and flexible link 66 are pushed out of the distal end 36 of the insertion tube 12, the flexible link 66 resumes its natural bent configuration as shown in Figure 2. The main imaging device 32 now captures a front-viewing image, and the auxiliary imaging device 64 simultaneously captures a rear-viewing image of the same area. The physician may then rotate the imaging assembly 14 so that the polarization planes of the polarizing filters 76,78 in the imaging assembly 14 are at a substantially 90° angle from the polarization planes of the polarizing filters 46, 48 in the polarizer cap 38. Once the correct orientation has been established, the physician locks or fixes the orientation of the imaging assembly 14 relative to the insertion tube 12. The physician then continues with the procedure.

The above-described embodiment is merely one of many alternative embodiments of the present invention. In one other alternate embodiment, polarizing filters are placed over only the auxiliary imaging device 64 of the imaging assembly 14 and the main light sources 34 to reduce light interference between them. In this embodiment, a low intensity auxiliary light source 70 may be used for the auxiliary imaging device 64 to alleviate any bright spots that could be seen by the main imaging device 32. This arrangement allows maximum light intake by the main imaging device 32 without light loss caused by a polarizing filter. Similarly, in another alternative embodiment, polarizing filters are placed over only the main imaging device 32 and the auxiliary imaging device 64. These two embodiments are useful depending on the types of imaging sensors used in the endoscope, specifically their light sensitivities, resistance to blooming, and dynamic ranges, as well as depending on the types of light sources used in the endoscope and their illumination intensities and/or wave length spectrums. In another alternate embodiment, the main imaging device 32 and main light sources 34 may share a polarizing filter. For example, the polarizer cap mounted on the distal end of the insertion tube may have a large polarizing filter approximately the size of the cap' end wall. This large filter may have openings for the channels 16 of the insertion tube 12. Alternatively, the filter may occupy only the area of the cap's end wall in front of the main imaging device 32 and main light sources 34. This embodiment allows for the orientation of the polarization plane in front of the main imaging device 32 and main light sources 34 to be precisely orientated.

In general, a polarizing filter may be placed in a cap mounted in front of an imaging device, placed in the lens assembly of the imaging device, or attached to the front of the imaging device. Various techniques for attachment may be used, such as ring and clamp arrangements, snap fit or plastic friction fit arrangements, or even permanent bonding. Alternatively, polarizing filters may be placed along the optical path of the fiber optic bundles that run along the length of the endoscope, or even placed in an external light source box.

In still another alternate embodiment, a cap with one or more polarizing filters, similar to the cap 38 shown in Figures 2 and 3, may be placed on the auxiliary imaging device 64.

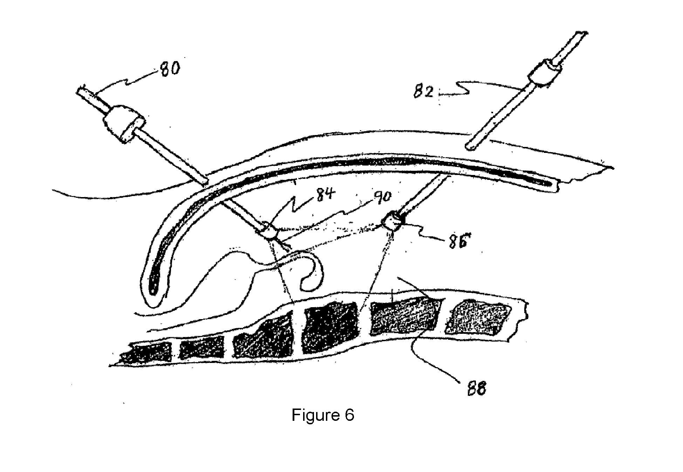

In yet another alternate embodiment, as shown in Figure 6, when multiple endoscopes 80, 82, which may be considered as an endoscope assembly, are used during a procedure, such as during laparoscopy or arthroscopy, a polarizer cap 84, 86 may be placed over one or more of the endoscopes 80, 82 to reduce light interference caused by endoscopes 80, 82. In Figure 6, the endoscopes 80, 82 are inserted into a peritoneal cavity 88. Preferably, at least one of the endoscopes 80, 82 has a channel that allows a surgical tool 90 to access the peritoneal cavity 88. In the illustrated embodiment, one or more of the endoscopes 80, 82 may have a light source. In some cases, each and every one of the endoscopes 80, 82 has a light source. Similarly, one or more of the endoscopes 80, 82 may have an imaging device. In some cases, each and every one of the endoscopes 80, 82 has an imaging device. In one particular case, only one of the endoscopes has a light source, while each of the other endoscopes has only an imaging device. In the illustrated embodiment, the polarizing filters of the same cap 84, 86 may have the same plane of polarization, and the polarizing filters of the different caps 84, 86 may have different planes of polarization. Alternatively, the caps may have the same plane of polarization but a physician can rotate one or more endoscopes to the appropriate orientations. If only two endoscopes 80, 82 are used, the two caps 84, 86 may have their planes of polarization orientated at 90° from one another. If only three endoscopes are used, the three caps may have polarization planes orientated at 120° relative to one another so as to provide a significant cancellation of light interference at each endoscope.

In a further alternate embodiment, the endoscope may have orientation arrangements on both the polarizer cap and the imaging assembly such that the orientation between polarizing filters may be easily adjusted. One such embodiment includes a small magnet that is permanently fixed to the polarizer cap, and a metallic element fixed to the imaging assembly. The magnet may be a rare earth magnet such as a Neodymium Iron Boron type permanent magnet. The orientation of the magnet and metallic element is designed such that the magnetic field generated by the magnet will attract the metallic element most strongly when the polarizing filters are properly aligned. In this manner, when the metallic element is in close proximity to the magnet, it will naturally be pulled by the magnet into the correct position. Furthermore, when the magnet and metallic element come into contact, an attachment is formed which resists change of orientation and maintains the proper orientation between the polarizing filters. Only when a substantial force is applied, such as when the physician forcefully advancing the imaging assembly, will the magnetic attachment be broken, allowing the physician to re-align or remove the imaging assembly. Alternatively, the magnet may be placed in the imaging assembly and a metallic element may be located on the cap. Furthermore, magnets may be used on both the imaging assembly and the cap.

In still a further alternate embodiment, the orientation features include a feature, such as a pin, rod or geometric feature, affixed to and protruding slightly away from the imaging assembly, and a feature, such as a cup and tube, on the polarizer cap that mates with the corresponding feature on the imaging assembly. The features may be made from a compressive material such as rubber so that when the two features are engaged a substantial force is needed to break the engagement. In this manner, the physician would first slide the imaging assembly past the distal end of the insertion tube and then, under the guidance of the auxiliary imaging device, rotate the imaging assembly to achieve the correct relative orientation between the polarizing filters. When the correct relative orientation between the polarizing filters is achieved, the physician may retract the imaging assembly so that the two features engage and lock together. To later disengage the features, the physician may forcefully advance the imaging assembly.

In yet a further alternate embodiment, the distal end of the imaging assembly includes a mechanism that can fix the position of the imaging assembly in the channel of the insertion tube. Such a mechanism may include the use of inflatable balloons, springs that are actuated via guide-wires, mechanical engagement arrangements, or factional methods such as large diameter compressive regions incorporating rubber or foam.

For reasons of completeness, various aspects of the present invention are set out in the following numbered clauses:
Clause 1. An endoscope assembly, comprising: an imaging device; a light source; and a polarizing filter disposed in front of the light source.
Clause 2. The endoscope assembly of clause 1, wherein the polarizing filter is attached to the light source.
Clause 3. The endoscope assembly of clause 1, further comprising a cap, wherein the polarizing filter is disposed on the cap.
Clause 4. The endoscope assembly of clause 3, further comprising a body having an end, wherein the imaging device and light source are disposed on the end of the body, and wherein the cap is mounted on the end of the body.
Clause 5. The endoscope assembly of clause 1, wherein the polarizing filter is a first polarizing filter, the endoscope assembly further comprising a second polarizing filter disposed in front of the imaging device, wherein each of the first and second polarizing filters has a plane of polarization, and wherein the planes of polarization of the first and second polarizing filters are aligned.
Clause 6. The endoscope assembly of clause 5, wherein the imaging device has a lens assembly, and wherein the second polarizing filter is disposed in the lens assembly.
Clause 7. The endoscope assembly of clause 6, wherein the light source is positioned to illuminate a field of view of the imaging device.
Clause 8. The endoscope assembly of clause 7, further comprising a cap, wherein the first and second polarizing filters are disposed on the cap.
Clause 9. The endoscope assembly of clause 8, further comprising a body having an end, wherein the imaging device and light source are disposed on the end of the body.
Clause 10. The endoscope assembly of clause 9, wherein the cap is mounted on the end of the body.
Clause 11. The endoscope assembly of clause 7, wherein the imaging device has a lens assembly, and wherein the second, polarizing filter is disposed in the lens assembly.
Clause 12. An endoscope assembly, comprising: an imaging device; a light source; and a polarizing filter disposed in front of the imaging device.
Clause 13. The endoscope assembly of clause 12, wherein the polarizing filter is attached to the imaging device.
Clause 14. The endoscope assembly of clause 12, further comprising a cap, wherein the polarizing filter is disposed on the cap.
Clause 15. The endoscope assembly of clause 14, further comprising a body having an end, wherein the imaging device and light source are disposed on the end of the body, and wherein the cap is mounted on the end of the body.
Clause 16. The endoscope assembly of clause 12, wherein the polarizing filter is a first polarizing filter, the endoscope assembly further comprising a second polarizing filter disposed in front of the light source, wherein each of the first and second polarizing filters has a plane of polarization, and wherein the planes of polarization of the first and second polarizing filters are aligned.
Clause 17. The endoscope assembly of clause 16, wherein the light source is positioned to illuminate a field of view of the imaging device.
Clause 18. An endoscope assembly, comprising: an imaging device; a light source; and a polarizing filter disposed in front of both the imaging device and the light source.
Clause 19. The endoscope assembly of clause 18, further comprising a cap, wherein the polarizing filter is disposed on the cap.
Clause 20. The endoscope assembly of clause 19, further comprising a body having an end, wherein the imaging device and light source are disposed on the end of the body.
Clause 21. The endoscope assembly of clause 20, wherein the cap is mounted on the end of the body.
Clause 22. The endoscope assembly of clause 18, wherein the light source is positioned to illuminate a field of view of the imaging device.
Clause 23. An endoscope assembly, comprising: an imaging device; a first polarizing filter disposed in front of the imaging device, wherein the first polarizing filter has a plane of polarization; a light source; and a second polarizing filter disposed in front of the light source, wherein the second polarizing filter has a plane of polarization, and wherein the plane of polarization of the first polarizing filter is at a substantially 90° angle from the plane of polarization of the second polarizing filter.
Clause 24. The endoscope assembly of clause 23, further comprising a cap, wherein the second polarizing filter is disposed on the cap.
Clause 25. The endoscope assembly of clause 24, further comprising a body having an end, wherein the imaging device is disposed on the end of the body.
Clause 26. The endoscope assembly of clause 25, wherein the cap is mounted on the end of the body.
Clause 27. The endoscope assembly of clause 23, wherein the imaging device has a lens assembly, and wherein the first polarizing filter is disposed in the lens assembly.
Clause 28. The endoscope assembly of clause 23, wherein the imaging device has an imaging sensor, and wherein the first polarizing filter is disposed in the imaging sensor.
Clause 29. The endoscope assembly of clause 23, wherein the light source faces the imaging device so that light from the light source is projected onto the imaging device.
Clause 30. The endoscope assembly of clause 23, wherein the imaging device is a first imaging device and the light source is a first light source, the endoscope assembly further comprising, a second imaging device; a third polarizing filter disposed in front of the second imaging device, wherein the third polarizing filter has a plane of polarization; a second light source; and a fourth polarizing filter disposed in front of the second light source, wherein the fourth polarizing filter has a plane of polarization, and wherein the plane of polarization of the third polarizing filter is at a substantially 90° angle from the plane of polarization of the fourth polarizing filter.
Clause 31. The endoscope assembly of clause 30, further comprising a cap, wherein the first and fourth polarizing filters are disposed on the cap.
Clause 32. The endoscope assembly of clause 31, further comprising a body having an end, wherein the first imaging device and the second light source are disposed on the end of the body.
Clause 33. The endoscope assembly of clause 32, wherein the cap is mounted on the end of the body.
Clause 34. The endoscope assembly of clause 30, wherein the first imaging device has a lens assembly, and wherein the first polarizing filter is disposed in the lens assembly.
Clause 35. The endoscope assembly of clause 30, wherein the first imaging device has an imaging sensor, and wherein the first polarizing filter is disposed in the imaging sensor.
Clause 36. The endoscope assembly of clause 30, wherein the first light source faces the first imaging device, and wherein the second light source faces the second imaging device.
Clause 37. The endoscope assembly of clause 36, wherein the second light source is positioned to illuminate a field of view of the first imaging device, and wherein the first light source is positioned to illuminate a field of view of the second imaging device.
Clause 38. The endoscope assembly of clause 37, further comprising: an insertion tube, wherein the first imaging device and second light source are mounted on the insertion tube; and an imaging assembly, wherein the second imaging device and first light source are mounted on the imaging assembly.
Clause 39. The endoscope assembly of clause 38, wherein the insertion tube having a channel, wherein a portion of the endoscope assembly is rotatably disposed inside the channel of the insertion tube, and wherein the second imaging device and first light source extend beyond an end of the insertion tube.
Clause 40. The endoscope assembly of clause 39, further comprising a locking mechanism that rotationally locks the insertion tube and imaging assembly.
Clause 41. The endoscope assembly of clause 36, further comprising: an insertion tube, wherein the first imaging device and second light source are mounted on the insertion tube; and an imaging assembly, wherein the second imaging device and first light source are mounted on the imaging assembly.
Clause 42. The endoscope assembly of clause 41, wherein the insertion tube having a channel, wherein a portion of the imaging assembly is rotatably disposed inside the channel of the insertion tube, and wherein the second imaging device and first light source extend beyond an end of the insertion tube.
Clause 43. The endoscope assembly of clause 42, further comprising a locking mechanism that rotationally locks the insertion tube and imaging assembly.
Clause 44. The endoscope assembly of clause 30, further comprising: an insertion tube, wherein the first imaging device and second light source are mounted on the insertion tube; and an imaging assembly, wherein the second imaging device and first light source are mounted on the imaging assembly.
Clause 45. The endoscope assembly of clause 44, wherein the insertion tube having a channel, wherein a portion of the imaging assembly is rotatably disposed inside the channel of the insertion tube, and wherein the second imaging device and first light source extend beyond an end of the insertion tube.
Clause 46. The endoscope assembly of clause 45, further comprising a locking mechanism that rotationally locks the insertion tube and imaging assembly.
Clause 47. The endoscope assembly of clause 23, further comprising: an insertion tube, wherein the light source is mounted on the insertion tube; and an imaging assembly, wherein the imaging device is mounted on the imaging assembly.
Clause 48. The endoscope assembly of clause 47, wherein the insertion tube having a channel, wherein a portion of the imaging assembly is rotatably disposed inside the channel of the insertion tube, and wherein the imaging device extends beyond an end of the insertion tube.
Clause 49. The endoscope assembly of clause 48, further comprising a locking mechanism that rotationally locks the insertion tube and imaging assembly.
Clause 50. An endoscope assembly, comprising: an insertion tube having a channel; an imaging assembly, wherein a portion of the imaging assembly is rotatably disposed inside the channel of the insertion tube; and a locking mechanism that rotationally locks the insertion tube and imaging assembly.
Clause 51. An major-minor endoscope comprising: a major endoscope having a first light source positioned proximate to one end and having a first imaging device positioned proximate to the one end, the major endoscope further including a channel along its length; a minor endoscope adapted for insertion into and through the channel of the major endoscope, the minor endoscope including an elongated body, a second light source positioned proximate to one end and having a second imaging device positioned proximate to the one end; a first polarizer positioned to polarize the light from the light source of one of the endoscopes; and a second polarizer positioned to polarize light received by the imaging device of the other endoscope.
Clause 52. An major-minor endoscope as in clause 51 wherein the first polarizer is positioned to polarize the light from the light source of the major endoscope and the second polarizer is positioned to polarize the light received by the imaging device of the minor endoscope.
Clause 53. An major-minor endoscope as in clause 51 wherein the first polarizer is positioned to polarize the light from the light source of the minor endoscope and the second polarizer is positioned to polarize the light received by the imaging device of the major endoscope.
Clause 54. An major-minor endoscope as in clause 53 further comprising a third polarizer positioned to polarize the light from the light source of the major endoscope and a fourth polarizer positioned to polarize the light received by the imaging device of the minor endoscope.
Clause 55. An endoscope for use with an independently positionable polarized light source, the endoscope comprising: an elongated body having a first imaging device positioned proximate to one end; and a first polarizer positioned to polarize light received by the imaging device.
Clause 56. An endoscope and independently positionable polarized light source comprising the endoscope of clause 55 and further comprising an independently positionable light source adapted for use within the human body, the light source including an elongated body, a first light source proximate to one end, and a polarizer positioned to polarize the light emitted from the light source.
Clause 57. A pair of polarized endoscopes adapted for cooperative use within the human body, wherein each endoscope, comprises an elongated body, an imaging device positioned proximate to one end, and a light source positioned proximate to the one end; wherein a polarizer is positioned to polarize the light from the light source of one of the endoscopes and a second polarizer is positioned to polarize the light received by the imaging device of the other endoscope.
Clause 58. A pair of endoscopes as in clause 57 further comprising a third polarizer positioned to polarize the light from the light source of the other endoscope and a fourth polarizer is positioned to polarize the light received by the imaging device of the one endoscope.

In the present application, the terms "insertion tube," "imaging assembly" and "endoscope" are interchangeable, may have the same or similar meanings, and may have the same or similar features and functions. Different terms are used in the application for ease of identification and description. Additionally, such a description should not be used to limit the breadth of the application. The use of "insertion tube," "imaging assembly", or "endoscope" merely refers to possible types of instruments in the broad field of endoscopy and the invention may be applied to many forms of endoscopes and medical imaging devices.

Furthermore, the configuration of one endoscope that is inserted through the channel of another endoscope (such as the imaging catheter being inserted through the main endoscope as described in the preferred embodiments) can be referred to as a major-minor endoscope configuration, where the larger diameter endoscope is referred to as the major endoscope and the smaller diameter endoscope as the minor endoscope.

## Claims

1. A major-minor endoscope assembly comprising:
a major endoscope having a first light source positioned proximate to one end and having a first imaging device positioned proximate to the one end, the major endoscope further including a channel along its length;
a minor endoscope adapted for insertion into and through the channel of the major endoscope, the minor endoscope including an elongated body, a second light source positioned proximate to one end and having a second imaging device positioned proximate to the one end;
a first polarizer positioned to polarize the light from the light source of one of the endoscopes; and
a second polarizer positioned to polarize light received by the imaging device of the other endoscope.

2. The major-minor endoscope assembly of claim 1, wherein the first polarizer is positioned to polarize the light from the light source of the major endoscope and the second polarizer is positioned to polarize the light received by the imaging device of the minor endoscope.

3. The major-minor endoscope assembly of claim 1, wherein the first polarizer is positioned to polarize the light from the light source of the minor endoscope and the second polarizer is positioned to polarize the light received by the imaging device of the major endoscope.

4. The major-minor endoscope assembly of claim 3, further comprising a third polarizer positioned to polarize the light from the light source of the major endoscope and a fourth polarizer positioned to polarize the light received by the imaging device of the minor endoscope.

5. The major-minor endoscope assembly of claim 4, further comprising a first cap, wherein the second polarizer and optionally the third polarizer are disposed on the first cap.

6. The major-minor endoscope assembly of claim 5, further comprising a second cap, wherein the first polarizer and optionally the fourth polarizer are disposed on the second cap.

7. The major-minor endoscope assembly of claim 5, wherein the first imaging device and the first light source are located at a distal portion of the major endoscope, and wherein the first cap is mounted on the distal end of the major endoscope and the optional second cap is mounted on the minor endoscope, optionally wherein the second cap is mounted on a distal end of the minor endoscope.

8. The major-minor endoscope assembly of claim 1 or 2, wherein the minor endoscope further comprises a lens assembly, and wherein the second polarizer is disposed in the lens assembly.

9. The major-minor endoscope assembly of any of the preceding claims, wherein the first and second polarizers are circular polarizing filters or linear polarizing filters.

10. The major-minor endoscope assembly of any of the preceding claims, wherein the second imaging device and second light source face the first imaging device and first light source, wherein the second polarizer blocks light that is transmitted through the first polarizer.

11. The major-minor endoscope assembly of any of the preceding claims, wherein the minor endoscope further comprises a flexible link that is at least partially made from a flexible shape memory material and is configured to assume a bent configuration when extending sufficiently from a distal end of the major endoscope such that the second imaging device and the second light source face the first imaging device and first light source.

12. The major-minor endoscope assembly of claim 10, wherein the first and second polarizers are linear polarizing filters, wherein the first polarizer has a first plane of polarization and the second polarizer has a second plane of polarization, wherein the first plane of polarization of the first polarizer is at a substantially 90° angle from the second plane of polarization of the second polarizer.

13. The major-minor endoscope assembly of any of the preceding claims, wherein the major endoscope further comprises a lens assembly, and wherein the first polarizer is optionally disposed in the lens assembly or in the first imaging device.

14. The major-minor endoscope assembly of claim 1, wherein a portion of the minor endoscope is rotatably disposed inside the channel of the major endoscope, wherein the second imaging device and the second light source extend beyond an end of the major endoscope channel, and optionally wherein a portion of the minor endoscope is slidably disposed inside the channel of the major endoscope, and optionally wherein the endoscope assembly further comprises a locking mechanism that locks the position of the minor endoscope with respect to the major endoscope.

15. The major-minor endoscope assembly of any of the preceding claims, wherein the major or minor endoscope optionally comprises at least one longitudinal channel through which surgical instruments and/or fluids may be passed and optionally wherein the major or minor endoscope is steerable.

16. The major-minor endoscope assembly of claim 5 or 6, wherein the first cap and/or second cap comprise magnets or orientation features configured to engage the first and/or second cap with the endoscope and/or imaging device.
